# EUROPEAN PATENT APPLICATION

(11) **EP 3 318 546 A1**
(43) Date of publication of application: **09.05.2018**
(21) Application number: 16197769.9
(22) Date of filing: 08.11.2016
(51) Int. Cl.: C07C 17/00, C07C 17/14, C07C 19/01, C07C 19/07, C07C 22/02, C07C 22/04, C07C 23/08, C07C 23/10, C07C 23/16, C07C 23/28, C07C 255/50, C07C 69/78, C07C 205/06, C07C 67/307, C07C 201/12, C07B 39/00, C07C 253/30

(54) **PROCESS FOR THE CHLORINATION AND IODINATION OF COMPOUNDS USING N-HYDROXYPHTHALIMIDE**

(71) Applicant: Justus-Liebig-Universität Gießen, 35390 Gießen (DE)
(72) Inventor: Schreiner, Peter R., 35435 Wettenberg (DE); Combe, Sascha H., 35080 Bad Endbach (DE)
(74) Representative: Stumpf, Peter

(57) **Abstract**

The invention provides a process for the production of halogenated or pseudo-halogenated hydrocarbons comprising the usage of at least one halogenating or pseudo-halogenating agent which contains at least one halogen-atom or pseudo-halogen substituent per formular unit in combination with substituted or unsubstituted *N-*hydroxyphthalimide and a metal-containing salt-like compound or a carboxylic or sulfonic acid with or without solvent.

## Description

### Field of the invention

The invention is about a chlorination process for chemical compounds. The chlorination preferably occurs at benzylic or aliphatic positions in hydrocarbon molecules.

### Background of the technology

Benzylic and alkyl chlorides are used for the production of a very large variety and quantity of chemicals. Well known methods for producing benzylic and alkyl chlorides are the thermal side-chain chlorination using elemental chlorine, as well as the usage of numerous other chlorinating agents, e.g., SO₂Cl₂, NaOCl, *^{t}*BuOCl, Et₄NH₄Cl, benzyltrimethylammonium tetrachloroiodate (BTME), 1,3-dichloro-5,5-dimethylhydantoin and NaCl/HCl, all of them allowing the chlorination of aliphatic C-H bonds. These radical-type chlorination reactions require heating or irradiation, and some chlorination agents are explosive, toxic as well as highly corrosive. The once-common solvent for side-chain chlorination reactions, CCl₄, is now forbidden in many countries due to its carcinogenic nature. Chlorination using elemental chlorine often results in nearly inseparable multiple side-chain chlorinated products. To avoid polychlorination nearly all of the methods mentioned above need a very large excess of substrate or, in many instances, the substrate is used as solvent, which represents a major drawback. For that reason the substrates typically are restricted to liquids. An additional drawback especially when using NaOCl or *^{t}*BuOCl is the formation of oxygenated products. Thus, controlled catalytic and safe chlorination reactions are not known in the state of the art.

### Content of the invention

It is therefore the aim of the present invention to address the aforementioned issues. This task is solved by the characterising technical features of claim 1; the dependent claims reveal additional preferred embodiments of the invention.

The invention revealed herein provides a safe, cheap, atom economic, air and moisture tolerating approach for the side-chain chlorination of a range of arenes and alkanes with yields of up to 85% at 25-30 °C with trichloroisocyanuric acid and *N*-hydroxyphthalimide **1** (NHPI) as radical initiator.

NHPI serves as a precursor for the phthalimido-*N*-oxyl **2** (PINO) radical (Scheme 1), which abstracts hydrogen atoms from C-H bonds and which is widely used in the oxidation of hydrocarbons, C-C as well as C-N couplings. PINO can be generated in the presence of some metal catalyst or a strong oxidant.

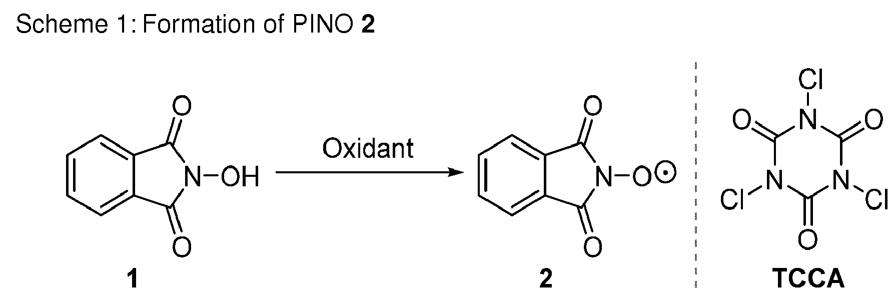

TCCA is produced at a scale of about 10⁶ tons per year; it is mainly used as a disinfectant and in food chemistry. All chlorine atoms in TCCA are typically utilized and it generally has a higher solubility than *N*-chlorosuccinimide (NCS), allowing highly concentrated reaction mixtures and reduction of the volume of organic solvent.

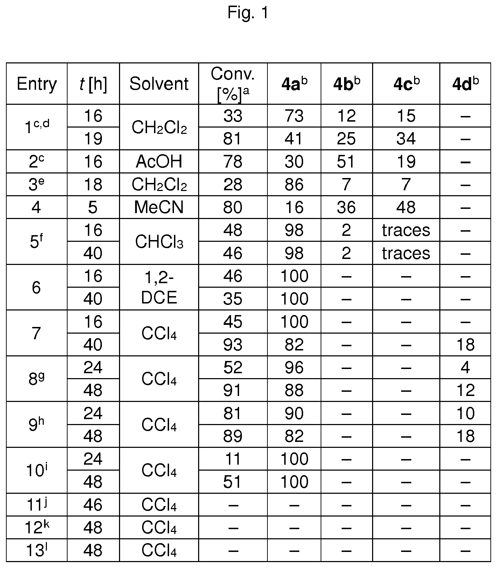

Table 1 (Fig. 1) shows the reaction conditions for the side-chain chlorination of toluene. Using CH₂Cl₂ as solvent affords 24% of benzyl chloride **4a** after 16 h (Table 1, entry 1). Adding two equivalents of CH₃OH to the reaction or using AcOH as solvent increases the amount of core substitution product (Table 1, entry 2). It is found that 0.4 equiv. of TCCA is enough for the reaction to proceed smoothly. Adding *tert*-butyl hydroperoxide (TBHP) does not accelerate the reaction (Table 1, entry 3). In MeCN mainly **4b** and **4c** are produced (Table 1, entry 4). Increasing the solvent polarity increases the ability of the system for aromatic substitution (Table 1, entry 4), however, the use of DMF or DMSO as solvent leads to a violent reaction without formation of products, probably due to reagent decomposition. In CHCl₃ and 1,2-dichloroethane the reaction ends at about 45% (Table 1, entries 5 and 6), even after adding more TCCA. Equally unfortunate, the best results are obtained in CCl₄, giving mainly benzyl chloride **4a** and some benzal chloride **4d** (Table 1, entry 7). Variation of the amount of Co(OAc)₂ (Table 1, entries 7-10) shows that 2 mol% give the best results (Table 1, entry 7). Higher concentrations of Co(OAc)₂ have an adverse effect on the outcome of the reaction leading to lower conversion even after long reaction times (Table 1, entry 10). The cobalt catalyst is crucial for this reaction and no product forms in the absence of Co(OAc)₂ (Table 1, entry 11). The use of NCS only leads to quantitative recovery of the starting materials, even at higher dilution (Table 1, entries 12-13). This may be due to its lower solubility or lower oxidation potential as compared to TCCA.

The assumption that catalytically active trihalomethyl radicals (here •CCl₃) are involved in the reaction is supported by performing the reaction in CH₂Cl₂ in the presence of CBr₄. Benzyl radicals can react with CBr₄ to form •CBr₃ radicals and the corresponding bromide. The formation of benzyl bromide **5** confirms the mechanistic suggestion (Table 2, entry 1, cf. scheme 2). Surprisingly it is observed that CBr₄ catalyzes the first stage of the reaction (Table 2, entries 1-2). Testing catalytic amounts of CBr₄ (Table 2, entries 3-7) shows that with 10 mol% CBr₄ the reaction almost completes after 16 h (Table 2, entry 5). Lower and higher CBr₄ loadings (Table 2, entries 3-4 and 6-7) as well as using HCBr₃ instead of CBr₄ (Table 2, entries 8-11) or HCCl₃ (Table 2, entry 12) give inferior results. The reaction does not proceed with less than 5 mol% of NHPI (Table 2, entries 13-14). An NHPI loading of 10 mol% (Table 2, entry 5) is sufficient and gives nearly the same result as with higher catalyst loadings (Table 2, entry 15). Even higher TCCA loadings drastically slow down the reaction most likely due to decreased solubility (Table 2, entries 16-17), which is in agreement with the results of Table 1 (entry 1). Remarkably, the reaction still proceeds in the presence of water albeit somewhat more slowly (Table 2, entries 18-19). The reaction is temperature dependent and is best performed at ambient temperature (25 °C) (Table 2, entries 20-21). Finally, it is also possible to substitute Co(OAc)₂ for less toxic Cu(OAc)₂ (Table 2, entry 22).

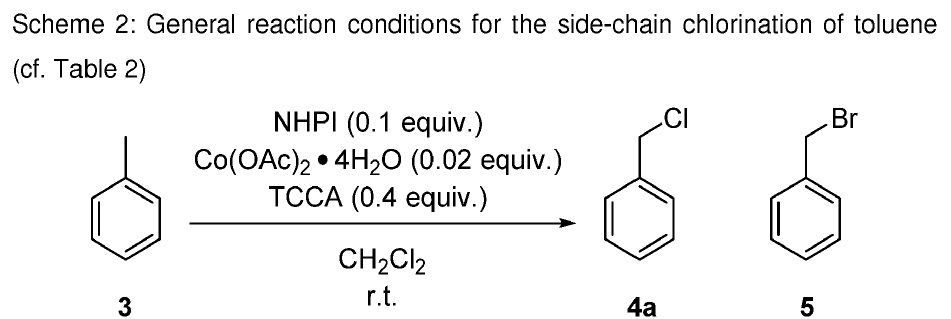

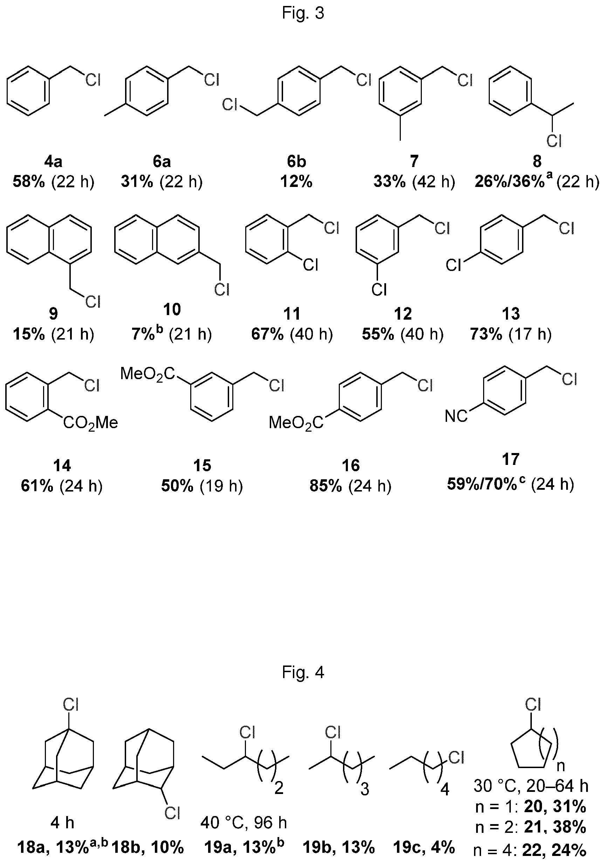

With the optimized reaction conditions (CBr₄ (0.125 mmol), CH₂Cl₂ (2 mL), Cu(OAC)₂•H₂O (0.025 mmol), NHPI (0.125 mmol), substrate (1.25 mmol), TCCA (0.5 mmol), 25 °C, cf. general method P1), the scope of the reaction is shown as follows (cf. Fig. 3). It is well known to the person skilled in the art, that the performed experiments as shown in the following paragraphs do not confine the scope of the invention to these examples.

In general, side-chain chlorination provides good yields for arenes carrying an electron-withdrawing (50-85%) and a weakly electron-donating group (31-73%); modest to strong electron donating groups favor the S_{E}Ar reaction. The stabilization of the resulting substituted tolyl radicals plays an important role concerning the yield, giving the following experimentally observed order: *p* > *o* > *m*, which is consistent with benzyl radical stabilization. Deactivating groups destabilize the radical, hence the substitution at the *meta*-position gives the lowest yield. Electron-withdrawing groups adjacent to the radical also have a destabilizing effect mostly in the proximity to the radical center. Consequently, highest yields are obtained for the para-products. Unlike *p*-tolunitrile, however, no reaction occurrs with *o*- and *m-*tolunitrile even when Co(OAc)₂ is used instead of Cu(OAc)₂. In the case of methyl 3-(chloromethyl)benzoate **15** the first direct side-chain chlorination is achieved. Structure **15** is an important building block in the synthesis of taprostene, a stable prostacyclin analogue used as vasodilator. The chlorination of ethylbenzene gives a 2°/1° selectivity of 10:1. Cumene reacts sluggishly and gives an inseparable reaction mixture of core and side-chain chlorinated products. Adamantane reacts very fast to 1-chloroadamantane **18a** and 2-chloroadamantane **18b** in a 5:1 ratio (GC-MS), which is comparable to Cl-radical mediated adamantane chlorinations. Surprisingly, no oxygenated products are obtained for any substrate examined. The formation of doubly chlorinated products can be diminished to traces or even avoided by reducing the reaction time and by using two equivalents of the substrate. Doubling the amount of the substrate also increases the product yield as exemplified with *p*-tolunitrile.

Also, the C-H bond chlorination of cyclic and acyclic alkanes on gram scale is investigated (Scheme 3). As bromocyclohexane is obtained with CBr₄ as catalyst the latter is omitted. A reason for this observation may lie - without confining to a certain theory - in the differences of the bond dissociation energies of the products. The bond dissociation energy (BDE) difference for the RCH₂-Br bond in benzyl bromide (BDE = 63 kcal mol⁻¹) and bromoethane (BDE = 72 kcal mol⁻¹) is about 9 kcal mol⁻¹. This energy difference seems to allow homolytic cleavage of benzyl bromide (Table 2, entry 1). 2-Bromopropane has a BDE of about 74 kcal mol⁻¹, i.e., bromocyclohexane is stable under the reaction conditions. Cyclic substrates with various ring sizes (Fig. 3) are chlorinated in moderate yields (24-38%) and can be purified by simple distillation. The chlorination of *n*-hexane gives a mixture of three regioisomers, preferentially 3-chlorohexane **19a** and 2-chlorohexane **19b** (3:1).

Not being confined to a certain theory, the mechanism of the inventive chlorination process may be described - according to the well known state of the art and the revelation contained herein (e.g., ab-initio-computations at the M06-2X/cc-pVTZ and B3LYP-D3/6-31 G(d,p) level of theory) -as follows (cf. Fig. 5).

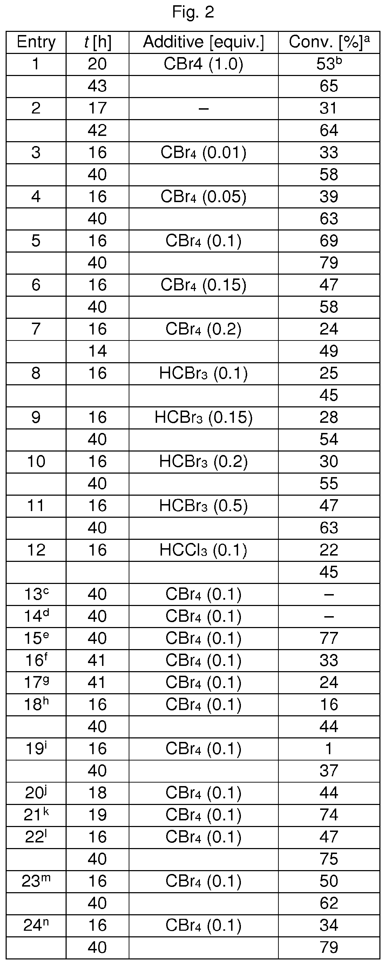

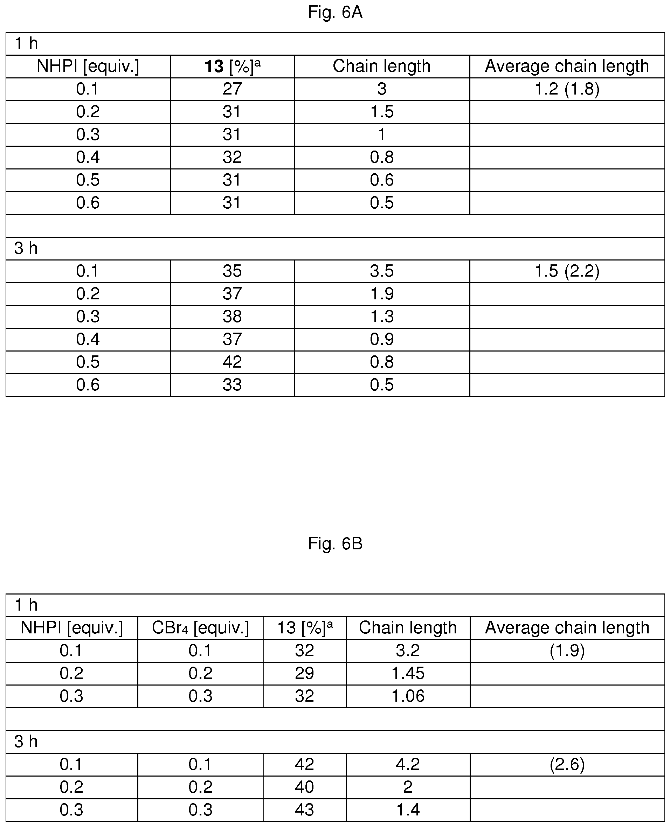

Cu(OAc)₂ and TCCA **23** generate the PINO radical **2** that abstracts an H• radical from **3** to form in an endergonic step (Δ*G*₂₉₈ = 11.9 kcal mol⁻¹) the corresponding benzyl radical **24** (Initiation). The chain length of NHPI is *one* (Fig. 6A), which means that NHPI serves only as radical initiator. Radical **24** subsequently reacts with **23** to form product **4a** as well as **25** (Δ*G*₂₉₈ = -15.8 kcal mol⁻¹). Radical **25** is also able to abstract an H• radical from **3** (Δ*G*₂₉₈ = -37.4 kcal mol⁻¹) and forms **26;** the latter reacts further until all chlorine is consumed and cyanuric acid **(27)** precipitates from the reaction mixture as a white solid (catalytic cycle **B**). The computed reaction enthalpies for the hydrogen abstraction from **3** with dichloroisocy-anuric acid radical **25,** monochlorisocyanuric acid radical, cyanuric acid radical (Δ*G*₂₉₈ = -26.1 to -27.6 kcal mol⁻¹) and •CBr₃ radical (Δ*G*₂₉₈ = -4.4 kcal mol⁻¹) show an overall highly exergonic process. The formation and precipitation of **27** is suggested to be the driving force of the reaction. CBr₄ catalyzes the reaction considerably (Fig. 2, entry 5) due to the favored formation of •CBr₃ radicals (Δ*G*₂₉₈ = - 9.3 kcal mol⁻¹). When all PINO is consumed trihalomethyl radicals carry on the chain reaction (Fig. 6B). Even if one equivalent of **24** is used to produce •CBr₃ radicals, homolytic cleavage of **5** (Fig. 2, entry 1) as well as C-H abstraction of **3** by a •CBr₃ radical gives overall two equivalents of **24** (catalytic cycle **A**).

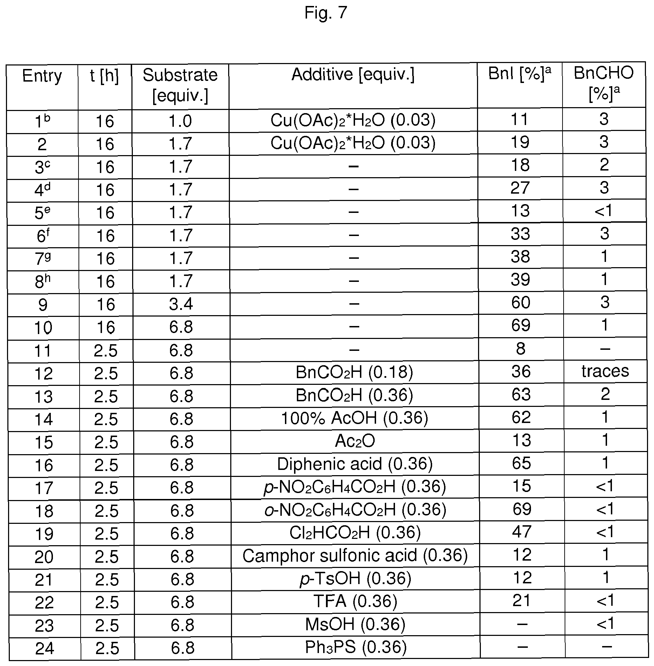

The wide scope of the inventive process is also shown by the investigations which were made regarding the iodination (cf. Fig 7 and scheme 3).

Using *N*-iodosuccinimide (NIS) in the reaction in place of TCCA gives benzyl iodide (Table 5, entry 1). DIH affords about twice as much product as compared to NIS (Table 5, entry 2). Remarkably, with DIH no metal catalyst is required and additionally a higher yield compared to NIS is obtained (Table 5, entry 6). DIH is a strong oxidant which is able to form PINO from NHPI. High dilution provides higher yields (Table 5, entries 3, 4, and 6). A NHPI loading of about 16 mol% is optimal for a smooth reaction (Table 5, entry 6). Lower NHPI loadings down to 8 mol% are lowering the yields (Table 5, entry 5) while higher loadings provide no additional benefit (Table 5 entries, 7 and 8). High substrate loadings also give higher yield (Table 5, entries 9 and 10). To activate the N-I bonds of DIH several carboxylic-and sulphonic acids are screened (Table 5, entries 12-23). In general, carboxylic acids afford much better yields than sulfonic acids, which can be correlated to their pKₐ values. Strong acids such as methane sulfonic acid lead to S*_{E}*Ar reactions.

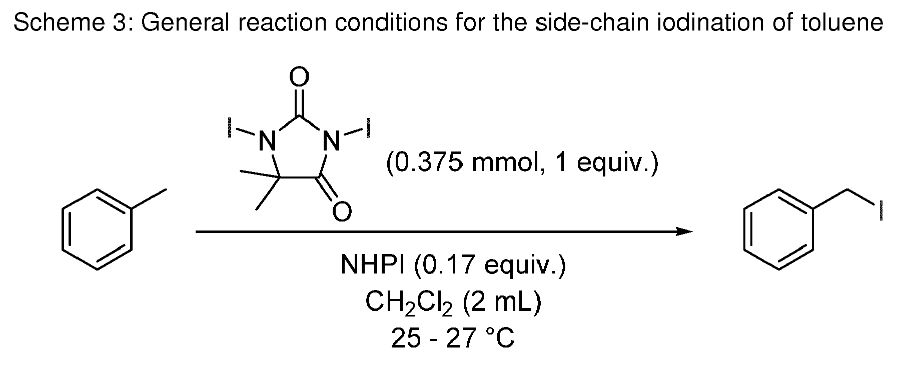

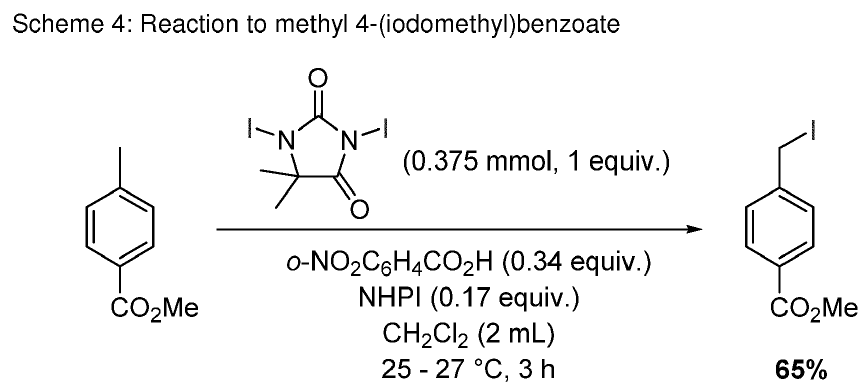

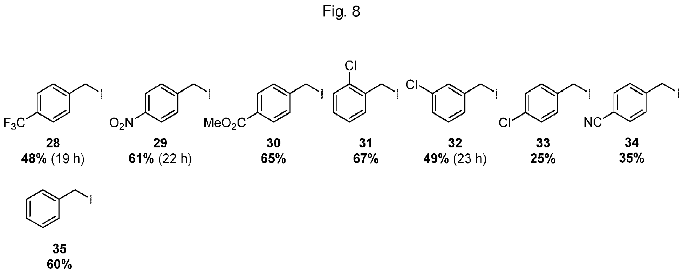

Fig. 8 shows some additional examples of iodine compounds accessible by use of the method revealed herein.

Thus, the invention provides a mild, safe, cheap, scalable, and controlled monohalogenation or mono pseudo-halogenation with only one equivalent of substrate and can be summarized as follows.

The invention is a process for the production of halogenated or pseudo-halogenated hydrocarbons using at least one halogenating or pseudo-halogenating agent which contains at least one halogen-atom or pseudo-halogen substituent per formular unit. It is generally characterized by the fact that at least one educt is reacted with at least one halogenating or pseudo-halogenating agent together with a substituted or unsubstituted *N*-hydroxyphthalimide and either a metal-containing salt-like compound or, alternatively, a carboxylic or sulfonic acid. The reaction mixture may additionally contain one or more solvents.

The halogenating agents according to the invention comprise at least one halogen atom per formular unit of either fluorine (F), chlorine (CI), bromine (Br) or iodine (I).

The at least one halogenating agent according to the invention is chosen from the list comprising *N*-chlorosuccinimide, substituted *N*-chlorosuccinimide, trichloroisocyanuric acid, 1,3-dichloro-hydantoin, substituted 1,3-dichloro-hydantoin, 1-chloro-hydantoin, substituted 1-chloro-hydantoin, *N*-bromosuccinimide, substituted *N-*bromosuccinimide, tribromoisocyanuric acid, 1,3-dibromo-hydantoin, substituted 1,3-dibromo-hydantoin, 1-bromo-hydantoin, substituted 1-bromo-hydantoin, *N-*iodosuccinimide, substituted *N*-iodosuccinimide, triiodoisocyanuric acid, 1,3-diiodo-hydantoin, substituted 1,3-diiodo-hydantoin, 1-iodo-hydantoin, substituted 1-iodo-hydantoin. It is well known to the person skilled in the art that by substituting the halogenating agent the reactivity of the halogenating agent can be adapted to the educt in order to optimize the product selectivity of the process. Thus the at least one substituent of the substituted *N*-chlorosuccinimide or substituted *N-*bromosuccinimide or substituted *N*-iodosuccinimide or substituted 1,3-dichloro-hydantoin or substituted 1-chloro-hydantoin or substituted 1,3-dibromo-hydantoin or substituted 1-bromo-hydantoin or substituted 1,3-diiodo-hydantoin or substituted 1-iodo-hydantoin can be chosen from the list comprising the substituents methyl, ethyl, propyl, isopropyl, n-butyl, *sec*-butyl, *tert*-butyl*,* phenyl or any other suitable substituent.

Especially the following substituted halogenating agents can be used within the scope of the invention: 1,3-dichloro-5,5-dimethylhydantoin, 1-chloro-3,5,5-trimethylhydantoin, 1-chloro-3,3,4-trimethylhydantoin, 1-chloro-5,5-dimethylhydantoin, 1-chloro-3,3-dimethylhydantoin, 1,3-dibromo-5,5-dimethylhydantoin, 1-bromo-3,5,5-trimethylhydantoin, 1-bromo-3,3,4-trimethylhydantoin, 1-bromo-5,5-dimethylhydantoin, 1-bromo-3,3-dimethylhydantoin, 1,3-diiodo-5,5-dimethylhydantoin, 1-iodo-3,5,5-trimethylhydantoin, 1-iodo-3,3,4-trimethylhydantoin, 1-iodo-5,5-dimethylhydantoin, 1-iodo-3,3-dimethylhydantoin. This list is, of course, in no way understood to be limiting the scope of the invention.

It is well known to the person skilled in the art that there are several chemical substituents existing which are chemically behaving similar to halogen atoms. For example, "CN" is forming a dimer, called "Dicyane" (NC-CN) analogously to F₂, Cl₂, Br₂ and I₂. Also, often the "-CN" substituent can be introduced into organic molecules analogously to the introduction of halogen atoms, for example via Sₙ2-Substitution of alcohols using sodium cyanide. Therefore substituents which are in some ways behave similar to halogens are called "pseudo-halogens". Thus it is obvious to the person skilled in the art that the invention, developed by use of halogenating agents, is also comprising the usage of a pseudo-halogenating agent. Examples of the at least one pseudo-halogen substituent per formular unit of the pseudo-halogenating agent are RS-, -CN, -SCN, -NCS, -OCN, -NCO, -CNO, -N₃,-SeCN, whereat this list is not limiting the invention to the mentioned pseudo-halogenic substituents and R is chosen from the list comprising -C₆H₅, -CH₃.

The at least one pseudo-halogenating agent which can be used according to the invention is chosen from the list comprising *N*-(phenylthio)phthalimide, substituted *N*-(phenylthio)phthalimide, *N*-(methylthio)phthalimide, substituted *N-*(methylthio)phthalimide, *N*-thiocyanate-succinimide, substituted *N*-thiocyanate-succinimide, whereat the at least one substituent of the substituted *N-*(phenylthio)phthalimide or substituted *N*-(methylthio)phthalimide or substituted *N-*thiocyanate-succinimide is chosen from the list comprising the substituents methyl, ethyl, propyl, isopropyl, n-butyl, sec-butyl, tert-butyl, phenyl, -OH, -OMe, -OEt, -NH₂, -NRₐR_{b}, -NO₂, -CO₂R_{c} whereat Rₐ, R_{b} and R_{c} are independently from each other chosen from the List comprising methyl, ethyl, propyl, i-propyl, butyl, sec-butyl, tert-butyl.

The invention further comprises a Process according to one of the previously described examples or a combination of at least two of the previously described examples, characterized in that the metal-containing salt-like compound contains at least one metal-atom chosen from the metals of the groups 6, 7, 8, 9, 10, 11 or 12 of the periodic system of chemical elements. As examples of metal-containing salt-like compounds according to the invention cobalt(II)acetate, Co(OAc)₂, or copper(II)acetate, Cu(OAc)₂ can be used.

The invention further comprises a Process where the carboxylic or sulfonic acid as revealed above exhibits a pKₐ value between -3 and 6, more preferred between -2 and 5, and most preferred between 3.5 and 1.2. Thus the carboxylic or sulfonic acid is preferably chosen from the list comprising the acids benzoic acid, acetic acid, diphenic acid, p-nitrobenzoic acid, o-nitrobenzoic acid, Camphor sulfonic acid, trifluoroacetic acid, p-toluenesulfonic acid. It is obvious to the person skilled in the art that this list is not understood to be confining the scope of the invention and that derivatives of carboxylic or sulfonic acids may also be applied, e.g., Acetic acid anhydride, halogenated acetic acid, e.g., diiodoacetic acid, dichloroacetic acid, trichloroacetic acid etc.

The substituted *N*-hydroxyphthalimide according to the invention contains at least one substituent chosen from the list comprising the substituents methyl, ethyl, propyl, isopropyl, n-butyl, *sec*-butyl, *tert*-butyl*,* phenyl, F, Cl, Br, I, CF₃, NO₂, CN, CO₂Me, OAc, NHR_{d}, NHAc, OH, OMe. R_{d} is chosen from the List comprising methyl, ethyl, propyl, *i*-propyl, butyl, *sec*-butyl, *tert*-butyl*.* It is obvious to the person skilled in the art that if the substituted *N*-hydroxyphthalimide contains two or more substituents, they are chosen independently from each other from the above mentioned list.

It is also possible to apply the substrate/educt in excess; the more substrate/educt is applied, the better the yield. Therefore the amount of substituted or unsubstituted *N*-hydroxyphthalimide within the reaction mixture may be set either with respect to educt or to halogenating agent/pseudo-haogenating agent. Thus the amount of substituted or unsubstituted *N*-hydroxyphthalimide lies between 0.1 mol% and 50 mol%, more preferred between 1 mol% and 40 mol%, even more preferred between 2 mol% and 30 mol% and most preferred between 3 mol% and 20 mol% in relation to the educt or the halogenating or pseudo-halogenating agent.

In the same way the reaction mixture may also contain a certain amount of CBr₄ between 0.1 mol% and 50 mol%, more preferred between 1 mol% and 30 mol%, even more preferred between 5 mol% and 20 mol% and most preferred between 7 mol% and 15 mol% in relation to the educt or the halogenating or pseudo-halogenating agent.

It is obvious to the person skilled in the art that the concentration of the metal containing salt-like compound resp. the carboxylic or sulfonic acid in the reaction mixture can vary to a great extent, depending on the individual activity of the compound resp. acid. It may range, e.g., from 0.2 mmol/mL until 200 mmol/mL or, more preferred, from 0.3 mmol/mL until 25 mmol/mL; larger or smaller concentrations may also be possible.

The invention finally also comprises the usage of the process as described above for the production of alkyl halides or benzyl halides or allyl halides or alkyl pseudo-halides or benzyl pseudo-halides or allyl pseudo-halides.

### Detailed embodiments of the invention

All chemicals used are purchased from TCI, Sigma Aldrich, and Alfa Aesar in reagent grade or better quality and are used without further purification. All solvents are distilled before usage with exception of 1,2-dichloroethane. Analytical thinlayer chromatography (TLC) was performed on SIL G UV₂₅₄ from Macherey Nagel with a fluorescence indicator. Visualization of the TLC plate was performed by UV (254 nm), 5% phosphomolybdic acid in CH₃CH₂OH or permanganate stain. Column chromatography is performed using Merck silica gel 60 (0.040-0.063 mm). ¹H and ¹³C spectra are measured with Bruker spectrometer Avance II 200 Hz (AV 200) and Avance II 400 MHz (AV 400), using TMS or the solvent peak as the internal standard. Reaction progress is monitored by GC-MS analysis with a Quadrupol-MS HP MSD 5971 (EI) and HP 5890A GC equipped with a J & W Scientific fused silica GC column (30 m x 0.250 mm, 0.25 micron DB-5MS stationary phase: 5% phenyl and 95% methyl silicone) using He (4.6 grade) as carrier gas; T-program standard 90-250 °C (10 °C/min heating rate), injector and transfer line 250 °C.

### General procedure P1 for the chlorination

In a 5 mL reaction vessel Cu(OAC)₂•H₂O (5.00 mg, 0.025 mmol, 0.02 equiv.), *N-*hydroxyphthalimide (20.4 mg, 0.125 mmol, 0.1 equiv.), CBr₄ (41.5 mg, 0.125 mmol, 0.1 equiv.) are diluted in CH₂Cl₂ (2 mL) and stirred for 0.5 min at 25 °C. Subsequently TCCA (116 mg, 0.500 mmol, 0.4 equiv.) and the substrate (1.25 mmol, 1.0 equiv.) is added and the vessel is sealed. After additional stirring for 17-22 h at 25 °C, the reaction mixture is diluted with sat. brine (10 mL), extracted with CH₂Cl₂ (3x10 mL), dried and concentrated under reduced pressure.

### General procedure P2 for the chlorination on gram scale without CBr₄

In a 250 mL reaction vessel Cu(OAC)₂•H₂O (0.232 g, 1.16 mmol, 0.02 equiv.), *N-*hydroxyphthalimide (0.946 g, 5.80 mmol, 0.1 equiv.) is diluted in CH₂Cl₂ (95 mL) and stirred for 0.5 min at 25 °C. Subsequently TCCA (5.39 g, 23.2 mmol, 0.4 equiv.) and the substrate (58.0 mmol, 1.0 equiv.) is added and the vessel is sealed. After additional stirring for 20-96 h at 25-30 °C, the reaction mixture is diluted with sat. brine (100 mL), extracted with CH₂Cl₂ (3x80 mL), dried and concentrated under reduced pressure.
**Benzyl chloride 4a:** (According to P1). The residue is purified by column chromatography (silica gel, 2 x 27 cm column, hexane/Et₂O 9:1) to give the chloride **4a** (91.9 mg, 0.726 mmol, 58% as a colorless liquid, R*_{f}* = 0.48; ¹H NMR (400 MHz, CDCl₃): *δ* = 7.41-7.30 (m, 5 H, CH_{Ar}), 4.60 (s, 2 H, CH₂) ppm; ¹³C NMR (100 MHz, CDCl₃): *δ* = 137.6 (C_{Ar}), 128.9 (CH_{Ar}), 128.7 (CH_{Ar}), 128.6 (CH_{Ar}), 46.4 (CH₂) ppm.
**4-methylbenzyl chloride 6a:** (According to P1). The residue is purified by column chromatography (silica gel, 2 x 27 cm column, hexane/Et₂O 19:1) to give the chloride **6a** (54.9 mg, 0.0.390 mmol, 31%) as a colorless liquid, R*_{f}* = 0.51; ¹H NMR (400 MHz, CDCl₃): *δ* = 7.29 (d, 2 H, *J =* 8.1 Hz, CH_{Ar}), 7.18 (d, 2 H, J = 7.8 Hz, CH_{Ar}), 4.58 (s, 2 H, CH₂), 2.37 (s, 3 H, CH₃) ppm; ¹³C NMR (100 MHz, CDCl₃): *δ* = 138.5 (C_{Ar}), 134.7 (C_{Ar}), 129.6 (CH_{Ar}), 128.7 (CH_{Ar}), 46.4 (CH₂), 21.3 (CH₃) ppm.
**1,4-Bis(chloromethyl)benzene 6b:** (According to P1). The residue is purified by column chromatography (silica gel, 2 x 27 cm column, hexane/Et₂O 19:1) to give the chloride **6b** (26.6 mg, 0.152 mmol, 12%) as a colorless solid, R*_{f}* = 0.35; m.p. 98-99 °C; ¹H NMR (400 MHz, CDCl₃): *δ* = 7.39 (s, 4 H, CH_{Ar}), 4.59 (s, 4 H, CH₂) ppm; ¹³C NMR (100 MHz, CDCl₃): *δ* = 137.8 (C_{Ar}), 129.1 (CH_{Ar}), 45.8 (CH₂) ppm.
**3-methylbenzyl chloride 7:** (According to P1). The residue is purified by column chromatography (silica gel, 2 x 27 cm column, hexane/Et₂O 19:1) to give the chloride 7 (58.4 mg, 0.415 mmol, 33%) as a colorless liquid, R*_{f}* = 0.48; ¹H NMR (400 MHz, CDCl₃): *δ* = 7.27-7.12 (m, 4 H, CH_{Ar}), 4.57 (s, 2 H, CH₂), 2.37 (s, 3 H, CH₃) ppm; ¹³C NMR (100 MHz, CDCl₃): *δ* = 138.6 (C_{Ar}), 137.5 (C_{Ar}), 129.5 (CH_{Ar}), 129.3 (CH_{Ar}), 128.8 (CH_{Ar}), 125.8 (CH_{Ar}), 46.5 (CH₂), 21.5 (CH₃) ppm.
**1-chloro-1-phenylethane 8:** (According to P1). The residue is purified by column chromatography (silica gel, 2 x 27 cm column, pentane) to give the chloride **8** (45.9 mg,0.327 mmol, 26%) as a colorless liquid, R*_{f}* = 0.29;
   According to P2: The residue is purified by distillation after 18 h to give a mixture of (2-chloroethyl)benzene (403 mg, 2.87 mmol, 5%) and chloro compound **21** (2.97 g, 21.1 mmol, 36%) as a colorless liquid, bp = 60 °C (10 mbar); yield determined by ¹H-NMR; ¹H NMR (400 MHz, CDCl₃): *δ* = 7.45-7.28 (m, 5 H, CH_{Ar}), 5.10 (q, 1 H, *J* = 6.8 Hz, CHCl), 2.83 (d, 3 H, *J* = 6.8 Hz, CH₃) ppm; ¹³C NMR (100 MHz, CDCl₃): *δ* = 142.8 (C_{Ar}), 128.6 (CH_{Ar}), 128.2 (CH_{Ar}), 126.5 (CH_{Ar}), 58.7 (CH), 26.5 (CH₃) ppm.
**1-(chloromethyl)naphthalene 9:** (According to P1). The residue is purified by column chromatography (silica gel, 2 x 27 cm column, hexane/Et₂O 19:1) to give the chloride **9** (34 mg, 0.192 mmol, 15%) as a light brownish viscous oil, R*_{f}* =0.18; ¹H NMR (400 MHz, CDCl₃): *δ* = 8.16 (d, 1 H, *J* = 8.4 Hz, CH_{Ar}), 7.91-7.84 (m, 2 H, CH_{Ar}), 7.63-7.58 (m, 1 H, CH_{Ar}), 7.56-7.51 (m, 2 H, CH_{Ar}), 7.46-7.41 (m, 1 H, CH_{Ar}), 5.07 (s, 2 H, CH₂) ppm; ¹³C NMR (100 MHz, CDCl₃): *δ* = 134.1 (C_{Ar}), 133.2 (C_{Ar}), 131.3 (C_{Ar}), 129.9 (CH_{Ar}), 129.0 (CH_{Ar}), 127.8 (CH_{Ar}), 126.9 (CH_{Ar}), 126.3 (CH_{Ar}), 125.4 (CH_{Ar}), 123.8 (CH_{Ar}), 44.7 (CH₂) ppm.
**2-chlorobenzyl chloride 11:** (According to P1). The residue is purified by column chromatography (silica gel, 2 x 27 cm column, hexane/Et₂O 12:1) to give the chloride 11 (136 mg, 0.842 mmol, 67%) as a colorless liquid, R*_{f}* = 0.48; ¹H NMR (400 MHz, CDCl₃): *δ* = 7.48-7.44 (m, 1 H, CH_{Ar}), 7.42-7.37 (m, 1 H, CH_{Ar}), 7.30-7.24 (m, 2 H, CH_{Ar}), 4.70 (s, 2 H, CH₂) ppm; ¹³C NMR (100 MHz, CDCl₃): *δ* = 135.2 (C_{Ar}), 134.2 (C_{Ar}), 131.0 (CH_{Ar}), 130.1 (CH_{Ar}), 130.0 (CH_{Ar}), 127.3 (CH_{Ar}), 43.7 (CH₂) ppm.
**3-chlorobenzyl chloride 12:** (According to P1). The residue is purified by column chromatography (silica gel, 2 x 27 cm column, hexane/Et₂O 12:1) to give the chloride **12** (110 mg, 0.684 mmol, 55%) as a colorless liquid, R*_{f}* = 0.46; ¹H NMR (400 MHz, CDCl₃): *δ* = 7.39 (bs, 1 H, CH_{Ar}), 7.33-7.24 (m, 3 H, CH_{Ar}), 4.54 (s, 2 H, CH₂) ppm; ¹³C NMR (100 MHz, CDCl₃): *δ* = 139.4 (C_{Ar}), 134.6 (C_{Ar}), 130.1 (CH_{Ar}), 128.8 (CH_{Ar}), 128.7 (CH_{Ar}), 126.8 (CH_{Ar}), 45.4 (CH₂) ppm.
**4-chlorobenzyl chloride 13:** (According to P1). The residue is purified by column chromatography (silica gel, 2 x 27 cm column, hexane/Et₂O 20:1) to give the chloride **13** (147 mg, 0.912 mmol, 73%) as a colorless solid, R*_{f}* = 0.37; m.p. 28-29 °C; ¹H NMR (400 MHz, CDCl₃): *δ* = 7.35-7.31 (m, 4 H, CH_{Ar}), 4.55 (s, 2 H, CH₂) ppm; ¹³C NMR (100 MHz, CDCl₃): *δ* = 136.1 (C_{Ar}), 134.4 (C_{Ar}), 130.1 (CH_{Ar}), 129.1 (CH_{Ar}), 45.5 (CH₂) ppm.
**Methyl 2-(chloromethyl)benzoate 14:** (According to P1). The residue is purified by column chromatography (silica gel, 2 x 27 cm column, hexane/Et₂O 9:1) to give the chloride **14** (141 mg, 0.764 mmol, 61%) as a colorless liquid, R*_{f}* = 0.20; ¹H NMR (400 MHz, CDCl₃): *δ* = 7.98 (d, 1 H, *J =* 7.8 Hz, CH_{Ar}), 7.60-7.50 (m, 2 H, CH_{Ar}), 7.43-7.37 (m, 1 H, CH_{Ar}), 5.05 (s, 2 H, CH₂), 3.94 (s, 3 H, CH₃) ppm; ¹³C NMR (100 MHz, CDCl₃): *δ* = 167.2 (CO), 138.9 (C_{Ar}), 132.7 (C_{Ar}), 131.2 (CH_{Ar}), 131.0 (CH_{Ar}), 129.2 (CH_{Ar}), 128.5 (CH_{Ar}), 52.4 (CH₂), 44.6 (CH₃) ppm.
**Methyl 3-(chloromethyl)benzoate 15:** (According to P1). The residue is purified by column chromatography (silica gel, 2 x 27 cm column, hexane/Et₂O 9:1) to give the chloride **15** (115 mg, 0.625 mmol, 50%) as a colorless liquid, R*_{f}* = 0.10; ¹H NMR (400 MHz, CDCl₃): δ = 8.06 (s, 1 H, CH_{Ar}), 7.99 (d, 1 H, *J =* 7.8 Hz, CH_{Ar}), 7.58 (d, 1 H, *J =* 7.6 Hz, CH_{Ar}), 7.43 (t, 1 H, *J =* 7.7 Hz, CH_{Ar}), 4.61 (s, 2 H, CH₂), 3.92 (s, 3 H, CH₃) ppm; ¹³C NMR (100 MHz, CDCl₃): *δ* = 166.6 (CO), 138.0 (C_{Ar}), 133.1 (C_{Ar}), 130.8 (CH_{Ar}), 129.8 (CH_{Ar}), 129.6 (CH_{Ar}), 129.0 (CH_{Ar}), 52.3 (CH₂), 45.6 (CH₃) ppm.
**Methyl 4-(chloromethyl)benzoate 16:** (According to P1). The residue is purified by column chromatography (silica gel, 2 x 27 cm column, hexane/Et₂O 9:1) to give the chloride **16** (196 mg, 1.06 mmol, 85%) as a colorless solid, R*_{f}* = 0.12; m.p. 36-37 °C; ¹H NMR (400 MHz, CDCl₃): *δ* = 8.03 (d, 2 H, *J=* 8.2 Hz, CH_{Ar}), 7.46 (d, 2 H, *J =* 8.2 Hz, CH_{Ar}), 4.61 (s, 2 H, CH₂), 3.92 (s, 3 H, CH₃) ppm; ¹³C-NMR (100 MHz, CDCl₃): *δ* = 166.7 (CO), 142.3 (C_{Ar}), 130.2 (C_{Ar}), 130.1 (CH_{Ar}), 128.6 (CH_{Ar}), 52.3 (CH₂), 45.5 (CH₃) ppm.
**4-(chloromethyl)benzonitrile 17:** (According to P1). The residue is purified by column chromatography (silica gel, 2 x 27 cm column, hexane/Et₂O 4:1) to give the chloride **17** (112 mg, 0.741 mmol, 59%)/(160 mg, 1.06 mmol, 70% (two equiv. of substrate used; yield related to TCCA)) as a colorless solid, R*_{f}* = 0.22; m.p, 80-81 °C; ¹H NMR (400 MHz, CDCl₃): *δ* = 7.66 (d, 2 H, *J=* 8.3 Hz, CH_{Ar}), 7.51 (d, 2 H, *J* = 8.3 Hz, CH_{Ar}), 4.60 (s, 2 H, CH₂) ppm; ¹³C NMR (100 MHz, CDCl₃): *δ* = 142.5 (C_{Ar}), 132.7 (CH_{Ar}), 129.3 (CH_{Ar}), 118.5 (C_{Ar}), 112.4 (C_{Ar}), 45.0 (CH₂) ppm.
**Chlorocyclopentane 20:** (According to P2). The residue is purified by distillation to give the chloro compound **20** (1.86 g, 17.7 mmol, 31%) as a colorless liquid; ¹H NMR (400 MHz, CDCl₃): *δ* = 4.44-4.33 (m, 1 H, CHCl), 2.05-1.81 (m, 6 H, CH₂), 1.73-1.58 (m, 2 H, CH₂) ppm; ¹³C NMR (50 MHz, CDCl₃): *δ* = 62.3 (CHCl), 37.2 (CH₂), 23.1 (CH₂) ppm.
**Chlorocyclohexane 21:** (According to P2). The residue is purified by distillation to give the chloro compound **21** (2.60 g, 22.2 mmol, 38%) as a colorless liquid, bp = 142 °C; ¹H NMR (200 MHz, CDCl₃): *δ* = 4.10-3.94 (m, 1 H, CHCl), 2.13-1.93 (m, 2 H, CH₂), 1.90-1.20 (m, 8 H, CH₂) ppm; ¹³C NMR (50 MHz, CDCl₃): *δ* = 60.5 (CHCl), 36.8 (CH₂), 25.3 (CH₂), 25.0 (CH₂) ppm.
**Chlorocyclooctane 22:** (According to P2). The residue is purified by distillation to give the chloro compound **22** (2.01 g, 13.8 mmol, 24%) as a colorless liquid, bp = 80 °C (10 mbar); ¹H NMR (400 MHz, CDCl₃): *δ* = 4.23 (sept, 1 H, CHCl), 2.16-2.06 (m, 2 H, CH₂), 2.05-1.91 (m, 2 H, CH₂), 1.83-1.68 (m, 2 H, CH₂), 1.65-1.42 (m, 8 H, CH₂) ppm; ¹³C NMR (50 MHz, CDCl₃): *δ* = 63.7 (CHCl), 35.3 (CH₂), 27.6 (CH₂), 25.1 (CH₂), 23.7 (CH₂) ppm.
**General procedure for the iodination (P3):** In a 5 mL reaction vessel *N-*hydroxyphthalimide (10.2 mg, 0.0625 mmol, 0.17 equiv.), o-nitro benzoic acid (22.7 mg, 0.136 mmol, 0.36 equiv.) are diluted in CH₂Cl₂ (2 mL) and stirred for 1 min at 25 °C. Subsequently DIH (143 mg, 0.375 mmol, 1 equiv.) and the substrate (2.50 mmol, 6.8 equiv.) is added and the vessel is sealed. After an additional stirring for 3 h at 25 °C, the reaction mixture is diluted with EtOAc (20 mL), washed with saturated NaHCO₃ (10 mL), Na₂S₂O₃ (5%, 10 mL), dried and concentrated under reduced pressure.
**4-(Trifluoromethyl)benzyl iodide 28:** (According to P3, 19 h). The residue is purified by column chromatography (silica gel, 2 x 27 cm column, hexane/EtOAc 9:1) to give the iodide **28** (79.2 mg, 0.277 mmol, 37%) as a colorless solid, R_{f} = 0.36; m.p. 44 °C; ¹H NMR (400 MHz, CDCl₃): *δ* = 7.56 (d, 2 H, J = 8.1 Hz, CH_{Ar}), 7.48 (m, 2 H, J = 8.1 Hz, CH_{Ar}), 4.46 (s, 2 H, CH₂) ppm; ¹³C NMR (100 MHz, CDCl₃): *δ* = 143.5 (d, J = 1.3 Hz), 130.1 (q, J = 33.6 Hz), 129.2, 126.0 (q, J = 3.8 Hz), 124.1 (q, J = 273.1 Hz), 3.31 (CH₂) ppm.
**4-Nitrobenzyl iodide 29:** (According to **P3,** 22 h). The residue is purified by column chromatography (silica gel, 2 x 27 cm column, hexane/EtOAc 9:1) to give the iodide **29** (121 mg, 0.459 mmol, 61%) as a colorless solid, R_{f} = 0.24; m.p. 127.4 °C; ¹H NMR (400 MHz, CDCl₃): *δ* = 8.16 (d, 2 H, J = 8.7 Hz, CH_{Ar}), 7.52 (m, 2 H, J = 8.7 Hz, CH_{Ar}), 4.48 (s, 2 H, CH₂) ppm; ¹³C NMR (100 MHz, CDCl₃): *δ* = 147.4, 146.9, 129.7, 124.3, 2.2 (CH₂) ppm.
**Methyl 4-(iodomethyl)benzoate 30:** (According to P3). The residue is purified by column chromatography (silica gel, 2 x 27 cm column, hexane/Et₂O 9:1) to give methyl 4-(iodomethyl)benzoate (134 mg, 0.485 mmol, 65%) as a colorless solid, R*_{f}* = 0.14; m.p. 72-73 °C; ¹H NMR (400 MHz, CDCl₃): *δ* = 7.98-7.94 (m, 2 H, CH_{Ar}), 7.45-7.41 (m, 2 H, CH_{Ar}), 4.46 (s, 2 H, CH₂), 3.91 (s, 3 H, CH₃) ppm; ¹³C-NMR (100 MHz, CDCl₃): *δ* = 166.7 (CO), 144.5 (C_{Ar}), 130.3 (C_{Ar}), 129.7 (CH_{Ar}), 128.9 (CH_{Ar}), 52.3 (CH₃), 4.0 (CH₂) ppm.
**2-chlorobenzyl iodide 31:** (According to P3). The residue is purified by column chromatography (silica gel, 2 x 27 cm column, hexane) to give the iodide **31** (127.0 mg, 0.503 mmol, 67%) as a colorless oil, R*_{f}* = 0.26; ¹H NMR (400 MHz, CDCl₃): *δ* = 7.44-7.38 (m, 1 H, CH_{Ar}), 7.37-7.32 (m, 1 H, CH_{Ar}), 7.24-7.18 (m, 2 H, CH_{Ar}), 4.53 (s, 2 H, CH₂) ppm; ¹³C NMR (100 MHz, CDCl₃): *δ* = 136.9, 134.0, 130.8, 130.3, 129.6, 127.5, 2.5 (CH₂) ppm.
**3-chlorobenzyl iodide 32:** (According to P3, 23 h). The residue is purified by column chromatography (silica gel, 2 x 27 cm column, hexane) to give the iodide **32** (93.0 mg, 0.368 mmol, 49%) as a colorless oil, R*_{f}* = 0.29; ¹H NMR (400 MHz, CDCl₃): *δ* = 7.37 (q, 1 H, *J =* 1.4 Hz, CH_{Ar}), 7.27-7.20 (m, 3 H, CH_{Ar}), 4.39 (s, 2 H, CH₂) ppm; ¹³C NMR (100 MHz, CDCl₃): *δ* = 141.4, 134.6, 130.2, 129.0, 128.2, 127.1, 3.74 (CH₂) ppm.
**4-chlorobenzyl iodide 33:** (According to P3, o-nitro benzoic acid (0.18 equiv.)). The residue is purified by column chromatography (silica gel, 2 x 27 cm column, hexane) to give the iodide **33** (46.3 mg, 0.183 mmol, 25%) as a colorless solid, R*_{f}* = 0.31; m.p. 60-61 °C; ¹H NMR (400 MHz, CDCl₃): *δ* = 7.34-7.30 (m, 2 H, CH_{Ar}), 7.29-7.25 (m, 2 H, CH_{Ar}), 4.43 (s, 2 H, CH₂) ppm; ¹³C NMR (100 MHz, CDCl₃): *δ* = 138.0, 133.8, 130.2, 129.2, 4.3 (CH₂) ppm.
**4-(chloromethyl)benzonitrile 34:** (According to P3). The residue is purified by column chromatography (silica gel, 2 x 27 cm column, hexane/EtOAc 9:1) to give the iodide **35** (64.6 mg, 0.266 mmol, 35%) as a colorless solid, R*_{f}* = 0.21; m.p. 140-142 °C; ¹H NMR (400 MHz, CDCl₃): *δ* = 7.59 (d, 2 H, *J* = 8.4 Hz, CH_{Ar}), 7.46 (d, 2 H, J=8.3 Hz, CH_{Ar}), 4.44 (s, 2 H, CH₂) ppm; ¹³C NMR (100 MHz, CDCl₃): *δ* = 144.8, 132.7, 129.6, 118.6, 111.7, 2.9 (CH₂) ppm.
**Benzyl iodide 35:** (According to P3). The residue is purified by column chromatography (silica gel, 2 x 27 cm column, hexane) to give the iodide **34** (99.0 mg, 0.454 mmol, 60%) as a colorless solid, R*_{f}* = 0.24; m.p. 24-25 °C ¹H NMR (400 MHz, CDCl₃): *δ* = 7.40-7.36 (m, 2 H, CH_{Ar}), 7.32-7.21 (m, 3 H, CH_{Ar}), 4.46 (s, 2 H, CH₂) ppm; ¹³C NMR (100 MHz, CDCl₃): *δ* = 139.5, 129.0, 128.9, 128.0, 5.8 (CH₂) ppm.

### Computations at the B3LYP-D3/6-31G(d,p)/CPCM level of theory

### (solvent: CH₂Cl₂)

All calculations based on Kohn-Sham density functional theory (DFT) are performed by means of the program package Gaussian09. Geometry optimizations are performed with the B3LYP. Empirical atom dispersion corrections by Grimme (D3) are used to treat non-covalent interactions. Frequency analysis is performed at the same level of theory to confirm that the optimized structures are local minima or transition states, and also to obtain the thermodynamic corrections to Gibbs free energy. Copper is treated by cc-PVDZ-DK all-electron basis set, while all remaining elements are represented by 6-31 G(d,p). The intrinsic reaction coordinate (IRC) calculations are performed to ensure that the obtained transition states connect the right reactants and products. Solution-phase (CPCM) single-point energy calculations are conducted on the basis of the gas-phase-optimized structures using dichloromethane as solvent at 298 K and 1 atm.

### Computations at the M06-2X/cc-pVTZ/CPCM level of theory (solvent: CH₂Cl₂)

All structures are optimized at the M06-2X/cc-pVTZ/CPCM (solvent: CH₂Cl₂) level of theory utilizing the Gaussian09 program package. The computed structures are characterized as minima on the potential energy hypersurface by vibrational frequency computations (with minima having only real frequencies) that also provide thermal corrections and zero-point vibrational energies (ZPVEs).

### Description of the drawings

- Fig. 1:: Table 1 - Reaction conditions for the side-chain chlorination of toluene. (^{a} Determined by GC-MS; ^{b} GC-MS ratios; ^{c} TCCA (1.0 equiv.); ^{d} After 16 h MeOH (2 equiv.) added; ^{e} TBHP (0.1 equiv.) added; ^{f} After 40 h additional TCCA (0.4 equiv.) added with no further conversion; ^{g} Co(OAC)₂•4H₂O (0.01 equiv.); ^{h} Co(OAC)₂•4H₂O (0.05 equiv.); ^{l} Co(OAC)₂•4H₂O (0.1 equiv.); ^{j} without Co(OAC)₂•4H₂O; ^{k} NCS (1.0 equiv.) instead of TCCA; ^{l}NCS (0.4 equiv.) instead of TCCA)
- Fig. 2:: Table 2 - Optimization of reaction conditions for the side-chain chlorination of toluene (^{a} Conversion of the starting material to **4a;** ^{b} Only here 5 is obtained after 20 h in the ratio 10 : 1 (**4a** : **5)** and disappears over the course of the reaction; ^{c} without NHPI; ^{d} NHPI (0.05 equiv.); ^{e} NHPI (0.2 equiv.); ^{f} TCCA (0.6 equiv.); ^{g} TCCA (0.8 equiv.); ^{h} H₂O (0.1 equiv.) added; ⁱ H₂O (0.3 equiv.) added; ^{j} 18 °C; ^{k} 25 °C; ^{l} Cu(OAc)₂•H₂O. ^{m} Cu(acac)₂. ⁿ CuCl₂.)
- Fig. 3:: Samples of Substrate scope for the side-chain chlorination of various toluene derivatives, yields of isolated pure products given. (^{a} According to general method P1; ^{b} Determined by ¹H NMR; ^{c} Two equiv. of substrate used)
- Fig. 4:: Samples of Substrate scope for the chlorination of hydrocarbons; yields of isolated pure products given. According to general method P2. (^{a} According to general method P1, without CBr₄; ^{b} Determined by ¹H-NMR)

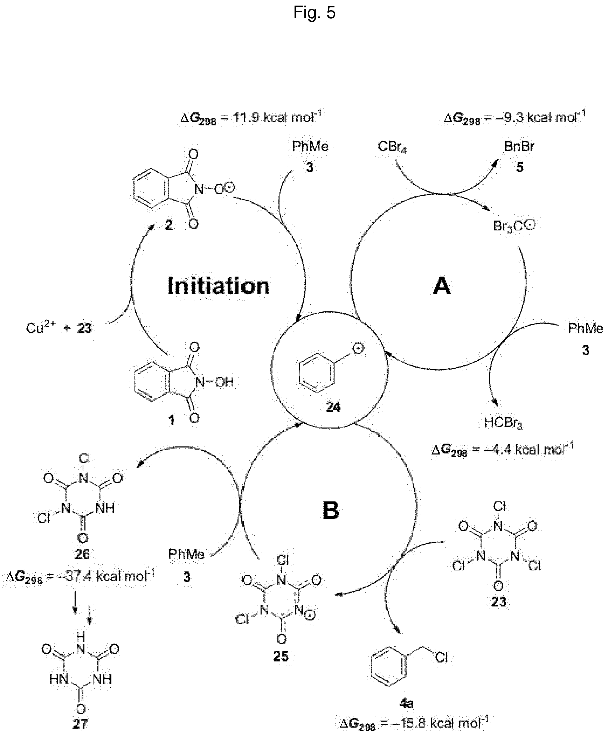

- Fig. 5:: Suggested mechanism for the side-chain chlorination of arenes, e.g. toluene
- Fig. 6A:: Table 3 - Radical chain length initiated by NHPI. (^{a} Yield determined by ¹H-NMR with dimethylterephthalate as internal standard.)
- Fig. 6B:: Table 4 - Radical chain length initiated by NHPI and CBr₄. (^{a} Yield determined by ¹H-NMR with dimethylterephthalate as internal standard.)
- Fig. 7:: Table 5 - Evaluation of reaction conditions for the side-chain iodination of toluene.
(^{a} Yield determined by ¹H-NMR with dimethyl terephthalate (DMT) as internal standard; ^{b} *N*-iodosuccinimide (0.625 mmol, 1 equiv.) used instead of DIH ^{c} 1 mL CH₂Cl₂; ^{d} 1.5 mL CH₂Cl₂ ; ^{e} 0.08 equiv. NHPI; ^{f} Average ¹H-NMR yield out of two runs; ^{g} 0.25 equiv. NHPI; ^{h} 0.33 equiv. NHPI)
- Fig. 8:: Substrate scope for the side-chain iodination of various toluene derivatives; yields of isolated pure products given. (Applied method: CH₂Cl₂ (2 mL), DIH (0.375 mmol), *o*-NO₂C₆H₄CO₂H (0.136 mmol), NHPI (0.068 mmol), substrate (2.50 mmol), 25-27 °C.

## Claims

1. Process for the production of halogenated or pseudo-halogenated hydrocarbons comprising the usage of at least one halogenating or pseudo-halogenating agent which contains at least one halogen-atom or pseudo-halogen substituent per formular unit, **characterized in that** at least one educt is reacted with the at least one halogenating or pseudo-halogenating agent together with substituted or unsubstituted *N*-hydroxyphthalimide and a metal-containing salt-like compound or a carboxylic or sulfonic acid, whereat the reaction mixture either contains at least one solvent or does not contain a solvent.

2. Process according to claim 1, **characterized in that** the at least one halogen atom per formular unit of the halogenating agent is chosen from the list containing F, Cl, Br, I.

3. Process according to claim 1 or claim 2, **characterized in that** the at least one halogenating agent is chosen from the list comprising *N*-chlorosuccinimide, substituted *N*-chlorosuccinimide, trichloroisocyanuric acid, 1,3-dichloro-hydantoin, substituted 1,3-dichloro-hydantoin, 1-chloro-hydantoin, substituted 1-chloro-hydantoin, *N*-bromosuccinimide, substituted *N*-bromosuccinimide, tribromoisocyanuric acid, 1,3-dibromo-hydantoin, substituted 1,3-dibromohydantoin, 1-bromo-hydantoin, substituted 1-bromo-hydantoin, *N-*iodosuccinimide, substituted *N*-iodosuccinimide, triiodoisocyanuric acid, 1,3-diiodo-hydantoin, substituted 1,3-diiodo-hydantoin, 1-iodo-hydantoin, substituted 1-iodo-hydantoin, whereat the at least one substituent of the substituted *N*-chlorosuccinimide or substituted *N*-bromosuccinimide or substituted *N-*iodosuccinimide or substituted 1,3-dichloro-hydantoin or substituted 1-chloro-hydantoin or substituted 1,3-dibromo-hydantoin or substituted 1-bromohydantoin or substituted 1,3-diiodo-hydantoin or substituted 1-iodo-hydantoin is chosen from the list comprising the substituents methyl, ethyl, propyl, *iso*propyl, n-butyl, *sec*-butyl, *tert*-butyl, phenyl.

4. Process according to claim 1 or claim 2, **characterized in that** the at least one halogenating agent is chosen from the list comprising 1,3-dichloro-5,5-dimethylhydantoin, 1-chloro-3,5,5-trimethylhydantoin, 1-chloro-3,3,4-trimethylhydantoin, 1-chloro-5,5-dimethylhydantoin, 1-chloro-3,3-dimethylhydantoin, 1,3-dibromo-5,5-dimethylhydantoin, 1-bromo-3,5,5-trimethylhydantoin, 1-bromo-3,3,4-trimethylhydantoin, 1-bromo-5,5-dimethylhydantoin, 1-bromo-3,3-dimethylhydantoin, 1,3-diiodo-5,5-dimethylhydantoin, 1-iodo-3,5,5-trimethylhydantoin, 1-iodo-3,3,4-trimethylhydantoin, 1-iodo-5,5-dimethylhydantoin, 1-iodo-3,3-dimethylhydantoin.

5. Process according to claim 1, **characterized in that** the at least one pseudo-halogen substituent per formular unit of the pseudo-halogenating agent is chosen from the list comprising RS-, -CN, -SCN, -NCS, -OCN, -NCO, -CNO, -N₃, -SeCN, whereat R is chosen from the list comprising -C₆H₅, -CH₃.

6. Process according to claim 5, **characterized in that** the at least one pseudo-halogenating agent is chosen from the list comprising *N-*(phenylthio)phthalimide, substituted *N*-(phenylthio)phthalimide, *N-*(methylthio)phthalimide, substituted *N*-(methylthio)phthalimide, *N-*thiocyanate-succinimide, substituted *N*-thiocyanate-succinimide, whereat the at least one substituent of the substituted *N*-(phenylthio)phthalimide or substituted *N*-(methylthio)phthalimide or substituted *N*-thiocyanate-succinimide is chosen from the list comprising the substituents methyl, ethyl, propyl, *iso*propyl, n-butyl, *sec*-butyl, *tert*-butyl*,* phenyl, -OH, -OMe, -OEt, -NH₂, -NRₐR_{b}, -NO₂, -CO₂R_{c} whereat Rₐ, R_{b} and R_{c} are independently from each other chosen from the List comprising methyl, ethyl, propyl, *i*-propyl, butyl, *sec*-butyl, *tert*-butyl.

7. Process according to one of the preceding claims or a combination of at least two of the preceeding claims, **characterized in that** the metal-containing salt-like compound contains at least one metal-atom chosen from the metals of the groups 6, 7, 8, 9, 10, 11 or 12 of the periodic system of chemical elements.

8. Process according to claim 7, **characterized in that** the metal-containing salt-like compound is either cobalt(II)acetate, Co(OAc)₂, or copper(II)acetate, Cu(OAC)₂.

9. Process according to one of the preceding claims 1 through 6 or a combination of at least two of the preceding claims 1 through 6, **characterized in that** the carboxylic or sulfonic acid exhibits a pKₐ value between -3 and 6, more preferred between -2 and 5, and most preferred between 3.5 and 1.2.

10. Process according to claim 9, **characterized in that** the carboxylic or sulfonic acid is chosen from the list comprising the acids benzoic acid, acetic acid, Diphenic acid, p-nitrobenzoic acid, o-nitrobenzoic acid, Camphor sulfonic acid, trifluoroacetic acid, p-toluenesulfonic acid.

11. Process according to any one of the preceding claims, **characterized in that** the substituted *N*-hydroxyphthalimide contains at least one substituent chosen from the list comprising the substituents methyl, ethyl, propyl, isopropyl, n-butyl, *sec*-butyl, *tert*-butyl*,* phenyl, F, Cl, Br, I, CF₃, NO₂, CN, CO₂Me, CO₂H, OAc, NHR_{d}, NHAc, OH, OMe, whereat R_{d} is chosen from the List comprising methyl, ethyl, propyl, *i*-propyl, butyl, *sec*-butyl, *tert*-butyl.

12. Process according to any one of the preceding claims, **characterized in that** the amount of substituted or unsubstituted *N*-hydroxyphthalimide lies between 0.1 mol% and 50 mol%, more preferred between 1 mol% and 40 mol%, even more preferred between 2 mol% and 30 mol% and most preferred between 3 mol% and 20 mol% in relation to the educt or the halogenating or pseudo-halogenating agent.

13. Process according to any one of the preceding claims, **characterized in that** the reaction mixture also contains a certain amount of CBr₄ between 0.1 mol% and 50 mol%, more preferred between 1 mol% and 30 mol%, even more preferred between 5 mol% and 20 mol% and most preferred between 7 mol% and 15 mol% in relation to the educt or the halogenating or pseudo-halogenating agent.

14. Process according to any one of the preceding claims, **characterized in that** the concentration of the metal containing salt-like compound or the carboxylic or sulfonic acid in the reaction mixture is between 0.2 mmol/mL and 200 mmol/mL, more preferred between 0.3 mmol/mL and 25 mmol/mL.

15. Usage of the process according to any one of the preceding claims for the production of alkyl halides or benzyl halides or allyl halides or alkyl pseudo-halides or benzyl pseudo-halides or allyl pseudo-halides.
